Europäisches Patentamt

European Patent Office

Office européen des brevets

(11) Veröffentlichungsnummer: **0 332 857**
**A1**

## (12) EUROPÄISCHE PATENTANMELDUNG

(21) Anmeldenummer: 89102491.1

(22) Anmeldetag: 14.02.89

(51) Int. Cl.⁴: **A61B 17/58**

(30) Priorität: 14.03.88 CH 953/88

(43) Veröffentlichungstag der Anmeldung:
20.09.89 Patentblatt 89/38

(84) Benannte Vertragsstaaten:
BE CH DE FR GB LI NL

(71) Anmelder: SYNTHES AG
Grabenstrasse 15
CH-7002 Chur(CH)

(72) Erfinder: Frigg, Robert
Obere Strasse 22
CH-7270 Davos-Platz(CH)
Erfinder: Heini, Paul Ferdinand
Eigerplatz 10
CH-3007 Bern(CH)

(74) Vertreter: Lusuardi, Werther Giovanni
Dr. Lusuardi AG Kreuzbühlstrasse 8
CH-8008 Zürich(CH)

(54) **Tibia-Marknagel zur Behandlung von Unterschenkelfrakturen.**

(57) Dieser einstückig ausgebildete Tibia-Marknagel weist gegen den Mittelteil (1) abgewinkelte proximale (2) und distale (3) Endteile auf. Das proximale Endteil (2) ist um den Winkel $\alpha = -20°$ und das distale Endteil (3) um den Winkel $\beta = +3°$ von der zentralen Achse (4) des Mittelteils (1) abgewinkelt. Das proximale Endteil (2) weist eine Länge A von 142 mm auf. Das proximale, sehr viel länger - im Vergleich zum Stand der Technik - ausgebildete Endteil (2) (Herzogkrümmung) taucht schon viel früher in die Markhöhle ein, und verhindert dadurch eine zu starke Durchbiegung oder Biegebeanspruchung des Marknagels. Das distale Endteil (3) dient beim Einschlagen des Marknagels auf der posterioren Kortikalis als Gleitschlitten.

Fig. 1

### Tibia-Marknagel zur Behandlung von Unterschenkelfrakturen

Die Erfindung bezieht sich auf einen einstückig ausgebildeten Tibia-Marknagel mit gegen den Mittelteil B abgewinkelten proximalen (A) und distalen (C) Endteilen.

Die Indikationen zur Verwendung von Tibia-Marknägeln sind neuerdings:
- Einfache Quer- oder Schrägfrakturen der Tibia;
- Etagenbrüche des Tibiaschaftes; und
- Trümmerbrüche des Tibiaschaftes.

Durch die erweiterte Indikation des Tibiamarknagels bei Unterschenkelfrakturen bestand ein Bedürfnis die Form und Art des Tibia-Marknagels zu ändern. Die Grundidee der Tibiamarknägel gemäss dem Stand der Technik war bis anhin den Knochen intern zu schienen. Dabei werden die Axialkraft, sowie die Torsionsmomente vom Knochen selber übernommen. Der Marknagel hat nur die Funktion die Knochenlängsachse aufrecht zu erhalten. Dadurch können relativ dünne, flexible Nägel verwendet werden, welche den Vorteil besitzen sich der Markhöhlenform anpassen zu können. Diese Anpassung ist vor allem beim Einbringen des Nagels wichtig, da der Eintrittspunkt des Marknagels nicht mit der Längsachse des Knochens fluchtet. Dieser Eintrittspunkt befindet sich an der Tuberositas Tibia leicht proximal des patellaren Sehnenansatzes. Nach Eröffnung der Markhöhle durch den Pfriem, und Aufbohren der Markhöhle wird der Marknagel eingeschlagen. Das Problem beim Einschlagen des Marknagels besteht darin, dass der Marknagel nach Durchquerung der Markhöhle auf der posterioren Kortikalis auftritt und nur durch ein elastisches oder plastisches Deformieren seiner Form weiter eingeschlagen werden kann. Bei hartem Knochen und jungen Patienten kann es vorkommen, dass die Marknagelspitze die posteriore Kortikalis durchstösst.

Um eine solche Perforation der posterioren Kortikalis zu verhindern, wurde die sogenannte Bündel-Nagelung (beispielsweise gemäss der DE-AS 23 41 439) vorgeschlagen. Bei dieser Methode werden ca. 2,0 mm dicke Drähte in die Markhöhle eingeschoben. Das hat den Vorteil, dass die einzelnen Drähte sehr flexibel sind und sich gut der Markhöhle anpassen können. Die Markhöhle wird bei dieser bekannten Methode mit einzelnen Drähten aufgefüllt, wobei eine Schienung des Knochens möglich wird. Diese Methode gemäss dem Stand der Technik hat sich bei der Behandlung von kurzen Schrägfrakturen im mittleren Schaftbereich der Tibia durchgesetzt. Die Nachteile dieser bekannten Methode sieht man jedoch dann, wenn eine Trümmerfraktur oder eine Etagenfraktur versorgt werden muss. Die einzelnen Drähte reichen dann nicht aus um eine Fraktur in axialer Richtung oder in Rotationsrichtung zu schützen. Zur Erhaltung der Knochenlänge, sowie zur Sicherung vor Rotationsbewegungen eignet sich nur ein in sich steifes und starres Implantat, das vorzugsweise eine sogenannte Verriegelung durch proximal und distal eingebrachte Querbolzen ermöglicht.

Die bis heute bekannten Tibia-Marknägel, die eine Axial-, sowie eine Rotations-Stabilität der Fraktur erzeugen, besitzen eine kurze proximale Abwinkelung (Herzogkrümmung), sowie einen geraden Teil der distal mit einer abgerundeten Spitze versehen wird, die das Auffädeln der Fragmente vereinfacht. Diese Nägel werden allerdings, wie oben beschrieben, bei deren Einführung relativ stark deformiert, so dass sich die Nägel plastisch in Längsrichtung verbiegen können.

Ist der Tibia-Nagel um 4/5 seiner Gesamtlänge eingeschlagen, taucht die Herzogkrümmung in die Tuberositas Tibia ein und entlastet somit die Biegebeanspruchung des Nagels. Diese Entlastung kann soweit gehen, dass bei voll eingeschlagenem Nagel, dieser lose in der Markhöhle zu liegen kommt.

Hier will die Erfindung Abhilfe schaffen. Der Erfindung liegt die Aufgabe zugrunde, einen Marknagel zu schaffen für die Behandlung von Unterschenkelfrakturen sämtlicher Indikationen mit einer optimal an die Anatomie der Markhöhle und der Implantationsmethodik angepassten Form. Insbesondere wird die Aufgabe gelöst einen Tibia-Marknagel zu schaffen, welcher einerseits ohne plastische Deformation in die Markhöhle eingeführt werden kann und anderseits nach Einführung eine stabile Schienung bewirkt.

Die Erfindung löst die gestellte Aufgabe mit einem Tibia-Marknagel,welcher die Merkmale des Anspruchs 1 aufweist.

Der erfindungsgemässe Tibia-Marknagel besitzt einen gegenüber der zentralen Achse des Mittelteils abgewinkelten distalen Endteil, der beim Einschlagen des Marknagels auf der posterioren Kortikalis als Gleitschlitten dient. Der Auftreffwinkel der Marknagelspitze ist dadurch spitzer und der Marknagel hat eine verstärkte Tendenz der Markhöhlengeometrie zu folgen. Das proximale, sehr viel länger - im Vergleich zum Stand der Technik - ausgebildete Endteil (Herzogkrümmung) taucht schon viel früher in die Markhöhle ein, und verhindert dadurch eine zu starke Durchbiegung oder Biegebeanspruchung des Marknagels. Die Dreipunktverspannung des Marknagels beim Einschlagen wird dadurch stark vermindert. Wird der Marknagel ganz eingeschlagen, legt sich diese überlange Herzogkrümmung der anterioren Kortikalis an. Das hat den Vorteil, dass die Tibia auch ohne Zusatzelemente am Nagel, optimal geschient wird.

Vergleicht man den erfindungsgemässen Marknagel mit den bekannten Nägeln gemäss dem Stand der Technik , so kann man feststellen, dass sich die bekannten Tibia-Marknägel beim Einschlagen verspannen, dann aber im eingeschlagenen Zustand, lose im Markraum zu liegen kommen. Dank seiner anatomisch angepassten Form kann der Marknagel gemäss der Erfindung relativ einfach und ohne übermässige Beanspruchung des Implantates oder des Knochens in die Markhöhle eingeführt werden und geht im eingeschlagenen Zustand eine formschlüssige Verbindung mit dem Knochen ein. Dieser grundlegende Unterschied wirkt sich noch stärker aus, wenn eine Verriegelungs-Marknagelung zu erfolgen hat, wobei das Implantat die Länge, sowie die Rotation des Knochens sichern muss. Diese sogenannten Verriegelungs-Marknägel besitzen eine erhöhte Wandstärke und dadurch eine ca. 80 % höhere Biegesteifigkeit. Um einen solchen Nagel implantieren zu können, war der Operateur bisher gezwungen die Markhöhle um 1,0 - 2,0 mm weiter aufzubohren als im Normalfall, mit dem Nachteil, dass relativ viel vitaler Knochen herausgefräst werden musste ohne eine erhöhte Stabilität der Osteosynthese zu erreichen.

Auch im Vergleich zur sogenannten Bündelungs-Marknagelung weist der erfindungsgemässe Nagel erhebliche Vorteile auf. Wie bereits erwähnt, handelt es sich bei der Bündelungs-Marknagelung um eine ausschliesslich innere Schienung des Röhrenknochens. Bei dieser Methode wird der meist unaufgebohrte Markraum mit ca. 2,0 mm dicken Drähten aufgefüllt. Die Qualität einer solchen Osteosynthese hängt stark vom Geschick des Chirurgen ab, da es nicht einfach ist diese Drähte in die Markhöhle einzuführen, ohne dass sich diese kreuzen und dadurch Engpässe in der Markhöhle schaffen, durch die kein Draht mehr geführt werden kann. Die Indikation dieser Nagelung ist sehr eng begrenzt, da die Markhöhle sanduhrenförmig ist und somit nur einen kurzen engen Teil aufweist, der mit Drähten genügend gefüllt und auf diese Weise stabilisiert werden kann. Die Möglichkeit der Erhaltung der Tibialänge bei Trümmerbrüchen oder bei Etagenbrüchen, sowie die Rotationssicherung bei dieser Art von Brüchen, ist bei der Bündelungs-Marknagelung nicht gegeben. Bei der Versorgung einer Etagenfraktur oder Trümmerfraktur, sowie einer relativ distal oder proximal gelegenen Fraktur, kann die Länge und auch die Rotation durch den erfindungsgemässen, der Anatomie optimal angepassten Tibia-Marknagel gesichert werden, da es sich dabei um ein relativ steifes Implantat handelt, welches die Belastung, die auf die Tibia wirkt, voll und ganz übernehmen kann.

Die auf die Tibia wirkenden axialen Kräfte, sowie die Torsionsmomente werden vorzugsweise über die sogenannten Verriegelungsschrauben auf den Marknagel übertragen. Diese Verriegelungsschrauben befinden sich proximal und distal der Frakturzone und garantieren so eine optimale Ruhigstellung der Fraktur.

Für die Aufnahme von Verriegelungsbolzen befinden sich vorzugsweise drei Löcher am proximalen Nagelende, die zweckmässigerweise in der frontalen Ebene plaziert sind. Das hat den Vorteil, dass die Bohrung für das Setzen der Schrauben von medial nach lateral erfolgt. Diese Orientierung der Schraube hat den Vorteil, dass keine Gefässe verletzt werden können. Die Ausrichtung der proximalen Verriegelungslöcher, 90° zur Längsachse, ermöglicht die Verwendung des gleichen Nagels für die linke wie für die rechte Tibia.

Die distale Verriegelung kann sowohl in der frontalen als auch in der sagittalen Ebene erfolgen. Bei sehr distal liegenden Frakturen ist die sagittale Verriegelung der frontalen vorzuziehen, da die Weichteildeckung der Schraubenköpfe zum Teil recht problematisch sein kann.

Bei der Verwendung von kurzen Tibia-Marknägeln, ist die Weichteildeckung in der sagittalen Ebene schwierig. Aus diesem Grund sollte die distale Verriegelung bei dieser Art von Verriegelung zweckmässigerweise von medial nach lateral erfolgen.

Ein Ausführungsbeispiel der Erfindung, welches zugleich das Funktionsprinzip erläutert, ist in der Zeichnung dargestellt und wird im folgenden näher beschrieben.

Fig. 1 stellt einen sagittalen Längsschnitt durch den erfindungsgemässen Tibia-Marknagel dar;

Fig. 2 stellt einen Schnitt längs der Linie II-II in Fig. 1 dar;

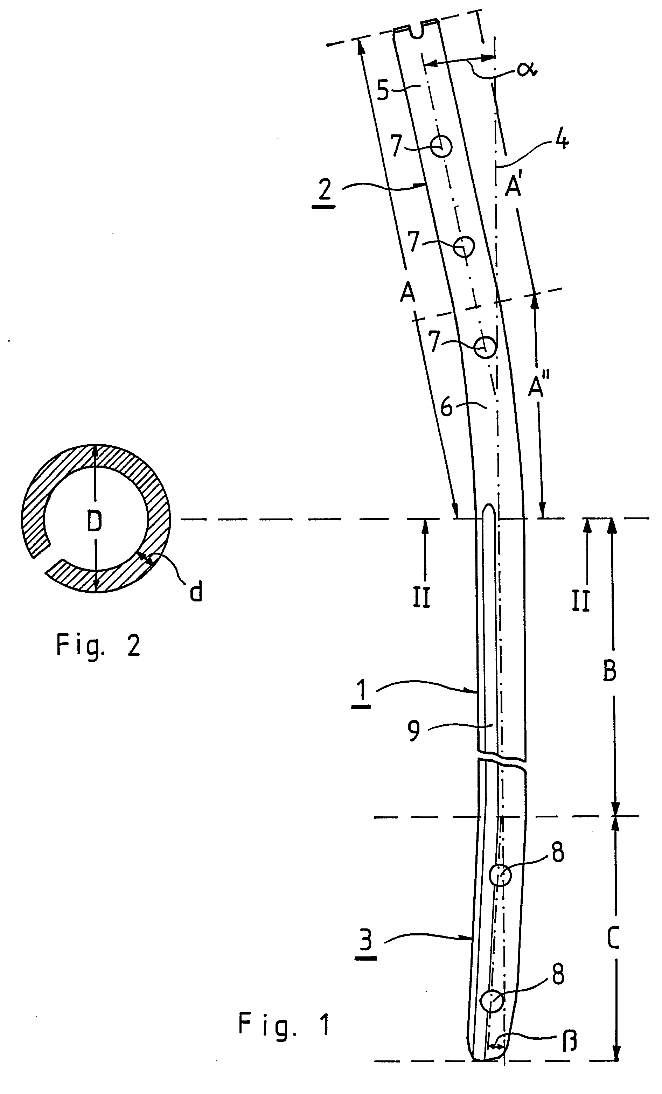

Fig. 1 und 2 zeigen einen rohrförmigen Tibia-Marknagel aus einem üblichen metallischen Implantatmaterial mit einem Aussendurchmesser D von 12 mm und einer Wandstärke d von 1,2 mm. Die Wandstärke d des hohlen Marknagels kann zwischen 0,9 und 1,3 mm, vorzugsweise zwischen 1,15 und 1,25 mm variieren. An das geradlinige Mittelteil 1 schliessen sich abgewinkelte proximale und distale Endteile 2 und 3. Das proximale Endteil 2 ist um einen Winkel $\alpha = -15°$ von der zentralen Achse 4 des Mittelteils 1 abgewinkelt und das distale Endteil 3 ist um den Winkel $\beta = +3°$ von der zentralen Achse 4 des Mittelteils 1 abgewinkelt. Der Winkel $\alpha$ kann innerhalb eines Bereiches von $-10° \leq \alpha \leq -20°$, vorzugsweise $-13° \leq \alpha \leq -17°$ variieren. Auch der Winkel $\beta$ kann innerhalb eines Bereiches von $+2° \leq \beta \leq +4°$ variieren.

Der proximale Endteil 2 besitzt eine Länge A von 142 mm und besteht aus einem endständigen geradlinigen Stück 5 der Länge A' = 80 mm und einem an den Mittelteil 1 anschliessenden gekrümmten Stück 6 der Länge A'' = 62 mm. Der Krümmungsradius r des an den Mittelteil 1 anschliessenden gekrümmten Stücks 6 beträgt 200 mm und kann im Bereich zwischen 180 und 220 mm, vorzugsweise zwischen 195 und 205 mm variiert werden.

Zu beachten ist, dass die Länge A des proximalen Endteils 2, bzw. die Teillängen A' und A'' der Teilstücke 5 und 6 auf eine Gesamtlänge des Marknagels L = A' + A'' + B + C von 220 mm abgestimmt sind. Für Marknägel mit einer anderen Gesamtlänge L - üblich sind Gesamtlängen bis zu 420 mm - werden die Teilstücke 5 und 6 zweckmässigerweise proportional verlängert. Im übrigen kann die Länge A des proximalen Endteils 2 auch bei einer Gesamtlänge L des Nagels von 220 mm innerhalb eines Bereiches von 122 - 162 mm, vorzugsweise von 137 - 147 mm variiert werden.

Das Längenverhältnis des endständigen geradlinigen Stück 5 zur Gesamtlänge L des Marknagels A':L kann im Bereich zwischen 0,33 und 0,40, vorzugsweise zwischen 0,350 und 0,377 variieren, währenddem das Längenverhältnis des gekrümmten Stücks 6 zur Gesamtlänge L des Marknagels A'': L im Bereich zwischen 0,25 und 0,31, vorzugsweise zwischen 0,270 und 0,294 variieren kann.

Im weiteren weist der Tibia-Marknagel im proximalen Endteil 2 drei Verriegelungslöcher 7 und im distalen Endteil 3 zwei Verriegelungslöcher 8 in der frontalen Ebene auf. Die Zahl der Verriegelungslöcher 7 und 8 kann je nach Verwendungszweck und Gesamtlänge des Marknagels variiert werden.

Schliesslich besitzt der erfindungsgemässe Tibia-Marknagel eine schlitzförmige Ausnehmung 9 in Richtung der zentralen Achse 4, welche sich durch den Mittelteil 1 bis zur Spitze des distalen Endteils 3 erstreckt.

## Ansprüche

1. Einstückig ausgebildeter Tibia-Marknagel mit gegen den Mittelteil (1) abgewinkelten proximalen (2) und distalen (3) Endteilen, dadurch gekennzeichnet, dass das proximale Endteil (2) um einen Winkel $-10° \leq \alpha \leq -20°$, vorzugsweise $-13° \leq \alpha \leq -17°$ von der zentralen Achse (4) des Mittelteils (1) abweicht und dass das distale Endteil (3) um den Winkel $+2° \leq \beta \leq +4°$ von der zentralen Achse (4) des Mittelteils (1) abweicht, und dass der proximale Endteil (2) eine Länge A von 122 - 162 mm, vorzugsweise von 137 - 147 mm aufweist.

2. Tibia-Marknagel nach Anspruch 1, dadurch gekennzeichnet, dass der proximale Endteil (2) aus einem endständigen geradlinigen Stück (5) der Länge A' und einem an den Mittelteil (1) anschliessenden gekrümmten Stück (6) der Länge A'' besteht.

3. Tibia-Marknagel nach Anspruch 1 oder 2, dadurch gekennzeichnet, dass das Längenverhältnis des endständigen geradlinigen Stück (5) zur Gesamtlänge L = A' + A'' + B + C des Marknagels A':L im Bereich zwischen 0,33 und 0,40, vorzugsweise zwischen 0,350 und 0,377 liegt.

4. Tibia-Marknagel nach einem der Ansprüche 1 bis 3, dadurch gekennzeichnet, dass das Längenverhältnis des gekrümmten Stücks (6) zur Gesamtlänge L = A' + A'' + B + C des Marknagels A'': L im Bereich zwischen 0,25 und 0,31, vorzugsweise zwischen 0,270 und 0,294 liegt.

5. Tibia-Marknagel nach einem der Ansprüche 1 bis 4, dadurch gekennzeichnet, dass das proximale Endteil (2) mindestens ein, vorzugsweise drei Verriegelungslöcher (7) aufweist.

6. Tibia-Marknagel nach einem der Ansprüche 1 bis 5, dadurch gekennzeichnet, dass das distale Endteil (3) mindestens ein, vorzugsweise zwei Verriegelungslöcher (8) aufweist.

7. Tibia-Marknagel nach Anspruch 5 oder 6, dadurch gekennzeichnet, dass die Verriegelungslöcher (7;8) in der frontalen Ebene plaziert sind.

8. Tibia-Marknagel nach einem der Ansprüche 1 bis 7, dadurch gekennzeichnet, dass die Wandstärke d des hohlen Marknagels zwischen 0,9 und 1,3 mm, vorzugsweise zwischen 1,15 und 1,25 mm liegt.

9. Tibia-Marknagel nach einem der Ansprüche 1 bis 8, dadurch gekennzeichnet, dass der Krümmungsradius r des an den Mittelteil (1) anschliessenden gekrümmten Stück (6) im Bereich zwischen 180 und 220 mm, vorzugsweise zwischen 195 und 205 mm liegt.

Fig. 2

Fig. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-C- 767 879 (E. POHL) <br> * Seite 2, Zeilen 19-24; Figur 1 * <br> --- | 1-7,9 | A 61 B 17/58 |
| Y | EP-A-0 091 499 (METALLWERK PLANSEE) <br> * Zusammenfassung * <br> --- | 1-7,9 | |
| Y | DE-A-3 537 318 (VEB KOMBINAT MEDIZIN UND LABORTECHNIK LEIPZIG) <br> * Seite 8, Zeile 28 - Seite 9, Zeile 5; Figuren 1a,1b * | 3 | |
| A | --- | 5,6 | |
| Y | DE-A-3 244 243 (H.G. ENDER) <br> * Figur 1 * <br> --- | 5-7 | |
| A | US-A-2 998 007 (K. HERZOG) <br> * Spalte 2, Zeilen 14-24; Spalte 4, Zeilen 6-19; Figuren 1,5 * <br> --- | 8 | |
| A | FR-A-2 387 637 (HOWMEDICA) <br> * Seite 4, Zeilen 16-34; Seite 5, Zeilen 2-6; Figur 3 * <br> --- | 1,5-7 | RECHERCHIERTE SACHGEBIETE (Int. Cl.4) |
| A | US-A-3 709 218 (W.X. HALLORAN) <br> * Spalte 6, Zeilen 7-18; Figuren 3,8 * <br> --- | 1,5,6 | A 61 B |
| A | US-A-4 169 470 (J. ENDER et al.) <br> * Spalte 7, Zeilen 24-68; Figuren 1,18 * <br> ----- | 1,2,4 | |

Der vorliegende Recherchenbericht wurde für alle Patentansprüche erstellt

| Recherchenort | Abschlußdatum der Recherche | Prüfer |
|---|---|---|
| DEN HAAG | 05-06-1989 | WOLF C.H.S. |